# EUROPÄISCHE PATENTSCHRIFT

(11) **EP 2 658 957 B1**
(45) Veröffentlichungstag und Bekanntmachung des Hinweises auf die Patenterteilung: **04.11.2015**
(21) Anmeldenummer: 11801993.4
(22) Anmeldetag: 23.11.2011
(51) Int. Cl.: C12M 1/26

(54) **HANDMESSGERÄT ZUM NACHWEIS VON VERDECKTEN SCHIMMELSCHÄDEN**
HANDHELD MEASURING DEVICE FOR DETECTING CONCEALED MOULD DAMAGE
APPAREIL DE MESURE PORTATIF POUR IDENTIFIER LA PRÉSENCE D'ATTAQUES DE MOISISSURES NON APPARENTES

(30) Priorität: 28.12.2010 DE 102010064251
(43) Veröffentlichungstag der Anmeldung: 06.11.2013
(73) Patentinhaber: Robert Bosch GmbH, 70442 Stuttgart (DE)
(72) Erfinder: NEFF, Petra, 70567 Stuttgart (DE)
(86) Internationale Anmeldenummer: PCT/EP2011/070726
(87) Internationale Veröffentlichungsnummer: WO 2012/089417

(56) Entgegenhaltungen:
- WO-A2-2007/130522
- "Operating Instructions AirPort MD8 AirSampler", Juni 2008 (2008-06), Sartorius Stedim Biotech, Göttingen Deutschland SM-606, XP002670810, Seiten 1-28, (S 4, Einleitung, Abs 2)
- M. FOTO ET AL: "A comparison of airborne ergosterol, glucan and Air-O-Cell data in relation to physical assessments of mold damage and some other parameters", INDOOR AIR, Bd. 15, Nr. 4, 1. August 2005 (2005-08-01) , Seiten 257-266, XP55020469, ISSN: 0905-6947, DOI: 10.1111/j.1600-0668.2005.00370.x
- Ashley P, Menkedick JR, Sell RN, Wooton MA, Weitz S, Gaitens J, Anderson J: "Healthy Homes Issues: Mold", März 2006 (2006-03), U.S. Department of Housing and Urban Development; Office of Healthy Homes and Lead Hazard Control, Washington D.C:, XP002670811, Seite 1-37, (S 12, Abs 2)(S 11, letzter Abs ff) (Tabelle 3, S 13)(S15, letzter Abs bis S 16, 2. Abs)
- MARTINEZ KENNETH F ET AL: "Exposure assessment and analysis for biological agents", GRANA, Bd. 43, Nr. 4, Dezember 2004 (2004-12), Seiten 193-208, XP002670812, ISSN: 0017-3134
- DOUWES J ET AL: "MEASUREMENT OF BETA(1 3)-GLUCANS IN OCCUPATIONAL AND HOME ENVIRONMENTS WITH AN INHIBITION ENZYME IMMUNOASSAY", APPLIED AND ENVIRONMENTAL MICROBIOLOGY, AMERICAN SOCIETY FOR MICROBIOLOGY, US, Bd. 62, Nr. 9, 1. September 1996 (1996-09-01), Seiten 3176-3182, XP001016212, ISSN: 0099-2240

## Beschreibung

Durch falsche Bauausführung, Wasserschäden oder fehlerhaftes Nutzungsverhalten kann in Innenräumen unerwünschtes Schimmelwachstum entstehen. Mögliche Gesundheitsstörungen, die insbesondere durch die Exposition mit den Schimmelsporen hervorgerufen werden können, sind Allergien, Infektionen oder auch Erkrankungen, die auf die Toxine zurückzuführen sind. Daher sollte die Schimmelbelastung in Innenräumen nach Möglichkeit ausgeschlosen werden.

Zum Nachweis verdeckten Schimmelbefalls werden bis dato verschiedene Methoden eingesetzt, die meistens auf mikrobiologischen Laboruntersuchungen basieren. So können z.B. Materialproben wie z. B. Tapete, Putz und Holz untersucht werden, wenn ein gezielter Verdacht auf eine Belastung vorliegt. Hierzu wird im Labor eine mikrobiologische Kultivierung der an den Material haftenden Schimmelpilze oder eine direkte mikroskopische Betrachtung der Materialien durchgeführt.

Ein weiterer Ansatz sind sogenannte Schnelltestverfahren, die vor Ort durchgeführt werden können. Dazu wird per Hand ein Abstrich mit Hilfe eines Wattestäbchens von gegebenenfalls belasteten Materialien aufgenommen, in einem weiteren manuellen Schritt in einer Testlösung extrahiert und anschließend durch Eintauchen eines Lateral-Flow-Teststreifen in die Testlösung auf Toxine untersucht. Sofern der Teststreifen sich verfärbt, deutet dies auf eine mögliche Belastung durch Schimmelpilze hin.

Ebenso wie die Untersuchung von Baumaterialien gehören Luftuntersuchungen zum Stand der Technik. Dazu wird z.B. mit Hilfe mikrobiologischer Luftprobennahmesysteme eine Probe gesammelt und anschließend im Labor auf Schimmelpilze bzw. deren flüchtigen Stoffwechselprodukte untersucht. Zu den in diesem Zusammenhang in der Entwicklung befindlichen Verfahren gehören z.B. die Testung auf Ergosterin oder beta-Glucane. Eine weiter Möglichkeit ist die gezielte Laboruntersuchung von Hausstaub.

Bei einer Probennahme aus der Luft bzw. dem Hausstaub ist die Unterscheidung wichtig, ob es sich um eine Innenraum- oder Außenquelle für die Pilze handelt. Dazu werden üblicherweise im Außenbereich Referenzproben genommen. Ein zusätzlicher Hinweis auf die Quelle des Schimmels stellt die Schimmelpilzart dar, da bestimmte Arten typisch für Feuchteschäden in Innenräumen sind und andere wiederrum typischerweise von außen eingetragen werden.

Neben den mikrobiologischen bzw. immunologischen Nachweismethoden, die gegebenenfalls auch mit fotometrischen Sensoren gekoppelt werden, stehen verschiedene elektrische Immunosensoren zur Verfügung, bei denen Moleküle oder Molekülkomplexe z.B. mittels Impedanzspektroskopie nachgewiesen werden können. Zu den neueren Verfahren in diesem Bereich gehört auch das in der DE102008027038 A1 beschriebene Redoxcycling, bei dem über die Zeit hinweg durch die wiederholte Reduktion und Oxidation der zum Nachweis notwendigen Moleküle der gebildete Strom erfasst und ggf. mit einer Elektrodenposition verglichen wird, an der oder in deren Nähe sich das Molekül nicht befindet.

### OFFENBARUNG DER ERFINDUNG

Gegenstand der vorliegenden Erfindung ist ein Handmessgerät zum Nachweis von verdeckten Schimmelschäden, insbesondere in Innenräumen, das zumindest folgende Komponenten aufweist:
- mindestens eine Sammeleinheit zur Aufnahme mindestens einer Innenraumprobe,
- mindestens eine Puffereinheit,
- mindestens eine immunologische Testeinheit,
- eine Auswertungseinheit, und
- ein Kontrollfeld.

Kern der Erfindung ist die Kombination einer Vorrichtung zur Probennahme mit einem immunologischen Schnelltest zu einem einfach zu bedienenden Handmessgerät, mit dem für Schimmelpilze charakteristische Antigene oder Haptene bestimmt werden können. Damit können sowohl solche Antigene oder Haptene nachgewiesen werden, die charakteristisch für alle Arten von Schimmelpilze sind, sowie solche Antigene oder Haptene, die spezifisch auf Pilzarten hinweisen, die typischerweise im Innenraum vorkommen. Um die gemessenen Werte mit Referenzwerten abgleichen zu können, kann das Gerät auch im Außenbereich angewendet werden, um so die Schimmelpilze, die typischerwesie nur im Innenbreich vorkommen, von denen im Außenbereich abzugrenzen. Liegt ein verdeckter Schimmelpilzbefall vor, können gegebenenfalls weitere Untersuchungen von Experten durchgeführt werden.

Ein Vorteil der Erfindung ist die einfache Handhabung, da nach Sammlung der Probe automatisch eine Aufnahme (Suspension, Lösung) der Probe in eine Pufferlösung erfolgt und diese Suspension oder Lösung automatisch dem immunologischen Test zugeführt wird. Hierzu sind keine weiteren manuellen Schritte notwendig. Auch das manuellen Zufügen von Pufferlösung zur Probe ist somit entbehrlich.

Ein weiterer Vorteil ist die kurze Testdauer. Das Ergebnis wird direkt auf dem Kontrollfeld innerhalb weniger Minuten angezeigt. Weitere Laboruntersuchungen, insbesondere mikrobiologischer Art sind nicht erforderlich. Mit dem Handmessgerät kann so schnell und einfach eine Aussage über eine verdeckte Schimmelbelastung getroffen werden. Darüber hinaus ist, je nach dem welche immunologische Testeinheit eingesetzt wird, eine qualitative und quantitative Aussage möglich.

Die Probe wird zunächst in einer Sammeleinheit des Handmessgerätes aufgenommen. Dazu können Bestandteile von Baumaterialien wie z.B. Tapeten, Putzstäube oder Ähnliches auf einen Träger innerhalb des Gerätes manuell appliziert werden. Bevorzugt wird jedoch eine Sammeleinheit nach dem Impaktions- und/oder Filtrationsverfahren. Dementsprechend weist die Sammeleinheit eine Prallplatte und/oder einen Filter auf. Bei der Prallplatte handelt es sich um ein Trägerplättchen, das mit einem Klebstoff zur Adhäsion der Schimmelpilzbestandteile beschichtet ist. Für die Filtration der Luft werden spezielle Filtermembranen eingesetzt, die sich zur Filtration von Schimmelpilzen bzw. deren Bestandteilen wie Sporen eignen. Dabei werden die sich in der Luft befindlichen Partikel mit einer Größe ab etwa 0,5 µm abgeschieden und auf der Filtermembran gesammelt. Um einen Luftstrom zu erzeugen, verfügt die Sammeleinheit über ein Luftansaugmodul, z.B. in Form einer Pumpe oder eines Ventilators. Der Luftansaugstrom beträgt ca. 100 l/min. Auch Stäube können auf diese Art aus der Luft abgeschieden und gesammelt werden. Um Kontaminationen zu vermeiden, wird die eigentliche Sammeleinheit, d.h. die Prallplatte bzw. der Filter als Einweg-Komponente in das Gerät eingelegt.

Der Sammeleinheit nachgeordnet ist eine Puffereinheit, in der sich eine Pufferkartusche befindet. Die gesammelte Probe wird vor der Analyse in die darin befindlichen Pufferlösung aufgenommen. In einer gesonderten Ausführungsform ist die Sammeleinheit und die Puffereinheit zu einer Einheit zusammengefasst, so dass das Sammeln der Probe und die Aufnahme in den Puffer zeitgleich erfolgt. Die Kartusche mit Pufferlösung kann ebenfalls als Einweg-Komponente ausgeführt sein und zusammen oder getrennt mit der Sammeleinheit in das Messgerät eingesetzt werden. Damit die Pufferlösung zur Sammelprobe gelangt, muß die Pufferkartusche zunächst geöffnet werden. Dies erfolgt entweder automatisch, sobald die Probennahme abgeschlossen ist, oder wird vom Anwender explizit ausgelöst. Um die Aufnahme und die Durchmischung der Probe in der Pufferlösung zu verbessern, kann ein separate Durchmischung erfolgen, die z.B. mit mechanischen Hilfsmitteln erfolgt. Hierzu ist zwischen der Puffereinheit und der immunologischen Testeinheit eine Einheit zur mechanischen Durchmischung der Probe angeordnet.

Die in die Pufferlösung aufgenommene Probe wird der immunologischen Testeinheit zugeführt. Alternativ werden die Probe und die Pufferlösung auch zeitgleich der Testeinheit zugeführt, so dass erst dort eine Durchmischung erfolgt. Je nach Ausführung des immunologischen Tests, weist die Testeinheit Komponenten zur Reagenzienzugabe, vorzugsweise eine Reagenzienkartusche, oder Lateral-Flow-Testkomponenten auf. Beim Lateral-Flow-Test erfolgt eine unmittelbare Bestimmung der Analyten. In einer alternativen Ausführungsform ist auch die Zugabe weiterer Testkomponenten mit Hilfe einer zusätzlichen Kartusche oder einer separat anzuschließenden Fluidik zur Bevorratung und dosierten Zugabe entsprechender Komponenten möglich.

Die immunologische Testeinheit wird als Einmaltest verwendet, so dass zu jeder Messung ein Austausch der Testkomponenten in Form eines Teststreifens und/oder einer Reagenzienkartusche notwendig ist. Alternativ kann die Testeinheit auch mehrfach verwendet werden, so dass ein Austausch nach jeder Einzelmessung entfällt.

Insgesamt können die Prallplatte und/oder der Filter, die Pufferkartusche, die Reagenzienkartusche oder die Lateral-Flow-Testkomponenten in unterschiedlicher Kombination oder einzeln als Einweg-Komponenten ausgeführt sein und entsprechend einzeln oder in Kombination in das Handmessgerät eingelegt bzw. angeschlossen werden.

Für den immunologischen Test kommen unterschiedliche Transduktionsprinzipien, z.B. in Form von optischen oder elektrischen Signalen, in Frage. Daher verfügt die Auswertungseinheit über ein fotometrisches oder elektronisches Transduktionsmodul. Die Funktionsweise hängt von dem jeweiligen Messprinzip ab. So ist z.B. eine fotometrische Analyse der Probe, aber auch eine Impedanzmessung oder eine fluorimetrische Messungmöglich. Generell ermöglicht das Gerät über einen oder mehrere Immuntests die parallele Bestimmung von mehreren Antigenen oder Haptenen. Hierzu stehen z.B. multianalytfähige Lateral-Flow-Tests (LFT) zur Verfügung. Die markierten Detektionsantikörper werden entweder bereits in der Pufferlösung vorgelegt oder sie befinden sich immobilisiert auf der Aufnahmefläche der Testeinheit. Sind entsprechende Antigene oder Haptene in der Probe vorhanden, binden die dadurch entstehenden Antigen-Antikörper-Konjugate an die entsprechenden Detektionsbereiche, die anschließend über die Auswertungseinheit optisch oder anderweitig ausgelesen werden. Es können solche Antigene oder Haptene bestimmt werden, die charakteristisch für Schimmelpilze im Allgemeinen sind wie z.B. Ergosterol oder beta-1,3-Glucan. Das Vorliegen dieser Substanzen weist darauf hin, dass in der Probe Pilze vorhanden sind. Desweiteren können solche Antigene oder Haptene bestimmt werden, die charakteristisch für spezielle Schimmelpilzarten sind, z.B bestimmte Oberflächenantigene von Schimmelpilzsporen. Solche differenzierten Analysen lassen Rückschlüsse zu, ob die Pilze mit großer Wahrscheinlichkeit von Außen eingetragen wurden oder ob sie aus einem Innenraumschaden stammen. Als für Innenraumbefälle charakteristischen Arten können z.B. die folgenden Pilzsporen gemessen werden: *Acremonium spp., Aspergillus penicillioides, Aspergillus restrictus, Aspergillus versicolor, Aureobasidium pullulans, Chaetomium spp., Phialophora spp., Scopulariopsis brevicaulis, Scopulariopsis fusca, Stachybotrys chartarum, Tritirachium (Engyodontium) album* oder *Trichoderma spp..*

Die Anzeige des Testergebnisses erfolgt in unterschiedlicher Form über ein Kontrollfeld. Bei einer fotometrischen Auswertung wird das Ergebnis unmittelbar anhand einer oder mehrerer charakteristischer Farbänderungen oder Verfärbungen, die gegebenenfalls im Abgleich mit einer Farbskala eine qualitative oder halbquantitative Aussage ermöglichen, abgelesen. So ist eine schnelle Aussage in Form von "nicht nachweisbar", "geringe Belastung" oder "hohe Belastung" möglich. Alternativ führt das Gerät intern eine optische oder elektrische Auswertung durch, welche als Ergebnis qualtitativ, halbquantitativ oder quantitativ durch eine elektronische Anzeige, z.B. über ein Display oder einzelne LEDs, angezeigt wird. Darüberhinaus verfügt das Gerät über eine Kontrollanzeige, welche die korrekte Ausführung des Test bestätigt oder gegebenenfalls auf mögliche Fehlerquellen hinweist.

Das Gerät kann sowohl mit Akkumulatoren als auch mit herkömmlichen Batterien betrieben werden.

In einem weiteren Aspekt betrifft die vorliegende Erfindung ein Verfahren zum Nachweis von verdeckten Schimmelschäden in Innenräumen mit dem zuvor beschriebenen Handmessgerät, das folgende Schritte umfasst:
a) Sammeln einer Innenraumprobe,
b) Aufnehmen der Probe in einem Puffer,
c) immunologische Testung der gepufferten Probe
d) Auswerten des immunologischen Tests durch elektronische oder optische Sensoren, und
e) Anzeigen des Testergebnisses aus Schritt d) in einem Kontrollfeld.

Dabei wird das Sammeln der Probe nach dem Impaktions- oder Filtrationsverfahren, d.h. mit Hilfe von adhäsiv beschichteten Prallplatten oder unter Einsatz von geeigneten Filtermembranen, durchgeführt. In einer bevorzugten Ausführungsform erfolgen das Sammeln der Probe und die Aufnahme der Probe in den Puffer bzw. die Pufferlösung zeitgleich, d.h. die Probe wird noch während des Sammelvorgangs im Puffer suspendiert. In einer weiteren Ausführungsform erfolgt die Aufnahme der Probe in den Puffer und die Bereitstellung für den immunologischen Test zeitgleich, d.h. die Probe und der Puffer gelangen parallel zur Aufnahmefläche des immunologischen Schnelltests und werden erst dort zusammengeführt. Sofern der Ablauf des Verfahrens in zeitlich nacheinander angeordneten Schritten abläuft, kann zwischen der Aufnahme der Probe in den Puffer und der immunologischen Testung ein Durchmischungsschritt erfolgen. Dabei wird die gepufferte Probe z.B. durch Vibration oder Rühren mechanisch durchmischt. In einer weiteren Ausführungsform des Verfahrens wird mit der immunologischen Testung in Schritt c) auf mindestens eine Komponente ausgewählt aus der Gruppe von: Ergosterol, beta-1,3-Glucan, und für Schimmelpilze artspezifische Marker, getestet.

Weitere Vorteile und vorteilhafte Ausgestaltungen der erfindungsgemäßen Vorrichtung und des erfindungsgemäßen Verfahrens werden durch die Abbildungen und Ausführungsbeispiele veranschaulicht und in der nachfolgenden Beschreibung erläutert. Dabei ist zu beachten, dass die Abbildungen und Ausführungsbeispiele nur beschreibenden Charakter haben und nicht dazu gedacht sind, die Erfindung in irgendeiner Form einzuschränken.

### Ausführungsbeispiel:

Das Handmessgerät beinhaltet eine Akku-betriebene Filtrationsvorrichtung mit einem Luftansaugstrom von ca. 100 l/min. Die sich im Luftstrom befindlichen Partikel > 0.5 µm werden durch eine geeignete Filtrationsmembran zurückgehalten und gesammelt. Der Filtrationsaufsatz besteht aus einer Ansaugdüse sowie einer Filtrationsmembran und ist zusammen mit einer Pufferkartusche und dem immunologischen Schnelltest als kunststoffbasierte Einwegkomponente integriert. Vor der Luftpartikelsammlung wird diese Wegwerfeinheit in das Handmessgerät eingelegt. Die Luftpartikelsammlung erfolgt über wenige Minuten. Im Anschluss wird per Knopfdruck die Kartusche mit Pufferlösung aufgebrochen. Die Pufferlösung gelangt dadurch auf die Filtrationsmembran und die gesammelten Partikel werden in der Lösung suspendiert. Durch einen weiteren Knopfdruck gelangt die suspendierte Probe zur Aufnahmefläche des immunologischen Schnelltests auf Basis eines multianalytfähigen Lateral Flow Tests (LFT). Die markierten Detektionsantikörper befinden sich immobilisiert auf der Aufnahmefläche. Bei Vorhandensein der zu detektierenden Analyten binden Antigen-Antikörper-Konjugate an die designierten Detektionsbereiche der Auswertungseinheit. Die optisch ausgelesenen Messwerte der verschiedenen Analyten, die dem Ergosterol-/ beta-1,3-Glucan-Gehalt entsprechen, werden auf einen Mikrokontroller übertragen, der auf Basis der gespeicherten Kalibrationsdaten sowie der angesaugten Luftmenge die Messwerte in die jeweilige Schimmelpilzsporenzahl pro Kubikmeter angesaugter Luft umrechnet. Um ein besserers quantitatives Ergebnis zu erhalten und den korrekten Testablauf zu prüfen, kann zusätzlich eine Referenzfläche der Filtermembran ausgewertet werden.

Die Ausgabe des Ergebnisses erfolgt auf einem Kontrollfeld bzw. Display. Darüber erhält der Anwender die Information ob überhaupt Pilze gefunden wurden, d.h. Ergosterol- bzw. beta-1,3-Glucan nachgewiesen werden konnte, und ob innenraumspezifische Arten gefunden wurden. Die Messwerte können entweder direkt als Sporenzahl pro Kubikmeter gesammelter Luft angegeben werden, oder bereits eine Bewertung durchlaufen. Dazu ist ein Bewertungsschema im Messgerät hinterlegt, mit welchem die Messwerte abgeglichen werden. Nach Ablauf der Messung oder vor der nächsten Messung wird die Einwegkomponente aus dem Handgerät entnommen.

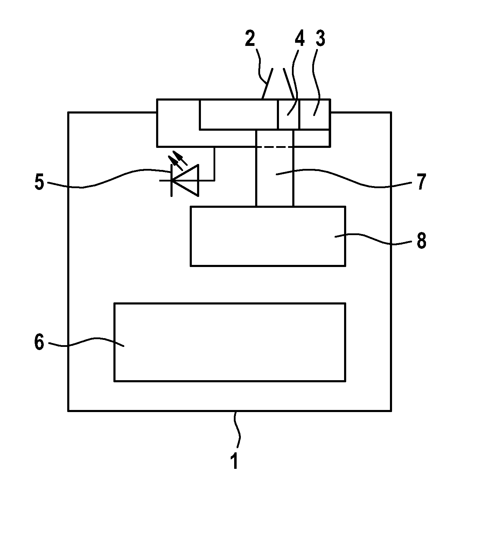

Fig. 1 zeigt eine schematische Ausführungsform des erfindungsgemäßen Handmessgerätes (1). Das Gerät umfasst eine Sammeleinheit (2) für Luft und Staub, zu der eine Pumpe oder ein Lüfter als Ansaugmodul (8) sowie ein Luft- bzw. Staubkanal (7) gehört. Kern der Sammeleinheit (2) ist jedoch der Impaktor oder Filter, auf der die in der Luft befindlichen Sporen der Schimmelpilze abgeschieden und gesammelt werden. Der Filter oder Impaktor ist als Einweg-Komponente ausgeführt. Die Pufferlösung befindet sich innerhalb der Puffereinheit (3) und ist in Form einer Einweg-Kartusche in das Handmessgerät (1) integriert. Die Schimmelsporen aus der Luft und dem Staub werden nach Abschluss des Sammelvorgangs in die Pufferlösung aufgenommen. Innerhalb der Testeinheit (4) ist der Einweg-Immunoassay für die Schimmelpilzsporen angeordnet. Die Auswertungseinheit (5) umfasst eine optische Auswerteoptik. Neben der notwendigen Auswertungselektronik weist das Handmessgerät (1) außerdem ein Kontrollfeld (6) bzw. Display auf, mit dem die Ergebnisse der Messung angezeigt werden.

## Patentansprüche

1. Handmessgerät (1) zum Nachweis von verdeckten Schimmelschäden, insbesondere in Innenräumen, **dadurch gekennzeichnet, dass** es zumindest folgende Komponenten aufweist:
- mindestens eine Sammeleinheit (2) zur Aufnahme mindestens einer Innenraumprobe,
- mindestens eine Puffereinheit (3),
- mindestens eine immunologische Testeinheit (4),
- eine Auswertungseinheit (5), und
- ein Kontrollfeld (6).

2. Handmessgerät (1) nach Anspruch 1, **dadurch gekennzeichnet, dass** die Sammeleinheit (2) eine Prallplatte und/oder einen Filter aufweist.

3. Handmessgerät (1) nach Anspruch 1 oder 2, **dadurch gekennzeichnet, dass** die Sammeleinheit (2) ein Luft- und/oder Staubansaugmodul (8) aufweist.

4. Handmessgerät (1) nach einem der vorhergehenden Ansprüche, **dadurch gekennzeichnet, dass** die Puffereinheit (3) eine Pufferkartusche aufweist.

5. Handmessgerät (1) nach einem der vorhergehenden Ansprüche, **dadurch gekennzeichnet, dass** die immunologische Testeinheit (4) Komponenten zur Reagenzienzugabe, vorzugsweise eine Reagenzienkartusche, oder Lateral-Flow-Testkomponenten aufweist.

6. Handmessgerät (1) nach einem der vorhergehenden Ansprüche, **dadurch gekennzeichnet, dass** die Auswertungseinheit (5) ein optisches oder elektronisches Transduktionsmodul aufweist.

7. Handmessgerät (1) nach einem der vorhergehenden Ansprüche, **dadurch gekennzeichnet, dass** die Prallplatte und/oder der Filter, die Pufferkartusche, die Reagenzienkartusche oder die Lateral-Flow-Testkomponenten in unterschiedlicher Kombination oder einzeln als Einweg-Komponenten ausgeführt sind.

8. Handmessgerät (1) nach einem der vorhergehenden Ansprüche, **dadurch gekennzeichnet, dass** die Sammeleinheit (2) und die Puffereinheit (3) zu einer Einheit zusammengefasst sind.

9. Handmessgerät (1) nach einem der vorhergehenden Ansprüche, **dadurch gekennzeichnet, dass** zwischen der Puffereinheit (3) und der immunologischen Testeinheit (4) eine Einheit zur mechanischen Durchmischung der Probe angeordnet ist.

10. Verfahren zum Nachweis von verdeckten Schimmelschäden, insbesondere Schimmelschäden in Innenräumen mit einem Handmessgerät nach einem der Ansprüche 1 bis 9,
**dadurch gekennzeichnet, dass** es zumindest folgende Verfahrensschritte umfasst:
a) Sammeln einer Innenraumprobe,
b) Aufnehmen der Probe in einem Puffer,
c) immunologische Testung der gepufferten Probe
d) Auswerten des immunologischen Tests durch elektronische oder optische Sensoren, und
e) Anzeigen des Testergebnisses aus Schritt d) in einem Kontrollfeld.

11. Verfahren nach Anspruch 10, **dadurch gekennzeichnet, dass** das Sammeln der Probe nach dem Impaktions- oder Filtrationsverfahren durchgeführt wird.

12. Verfahren nach Anspruch 10 oder 11, **dadurch gekennzeichnet, dass** Schritt a) und b), oder Schritt b) und c) zeitgleich durchgeführt werden.

13. Verfahren nach einem der Ansprüche 10 bis 12, **dadurch gekennzeichnet, dass** die in Schritt b) in einen Puffer aufgenommene Probe vor der immunologischen Testung in Schritt c) durchmischt wird.

14. Verfahren nach einem der Ansprüche 10 bis 13, **dadurch gekennzeichnet, dass** mit der immunologischen Testung in Schritt c) auf mindestens eine Komponente ausgewählt aus der Gruppe von: Ergosterol, beta-1,3-Glucan, und für Schimmelpilze artspezifische Marker, getestet wird.

## Claims

1. Handheld measuring device (1) for detecting concealed mould damage, in particular in interiors, **characterized in that** it has at least the following components:
- at least one collecting unit (2) for accommodating at least one interior sample,
- at least one buffer unit (3),
- at least one immunological test unit (4),
- an evaluation unit (5), and
- a control panel (6).

2. Handheld measuring device (1) according to Claim 1, **characterized in that** the collecting unit (2) has a baffle plate and/or a filter.

3. Handheld measuring device (1) according to Claim 1 or 2, **characterized in that** the collecting unit (2) has an air and/or dust intake module (8).

4. Handheld measuring device (1) according to one of the preceding claims, **characterized in that** the buffer unit (3) has a buffer cartridge.

5. Handheld measuring device (1) according to one of the preceding claims, **characterized in that** the immunological test unit (4) has components for adding reagents, preferably a reagent cartridge, or lateral flow test components.

6. Handheld measuring device (1) according to one of the preceding claims, **characterized in that** the evaluation unit (5) has an optical or electronic transducer module.

7. Handheld measuring device (1) according to one of the preceding claims, **characterized in that** the baffle plate and/or the filter, the buffer cartridge, the reagent cartridge or the lateral flow test components are embodied in different combination or individually as disposable components.

8. Handheld measuring device (1) according to one of the preceding claims, **characterized in that** the collecting unit (2) and the buffer unit (3) are combined to form one unit.

9. Handheld measuring device (1) according to one of the preceding claims, **characterized in that** a unit for thorough mechanical mixing of the sample is arranged between the buffer unit (3) and the immunological test unit (4).

10. Method for detecting concealed mould damage, in particular mould damage in interiors with the aid of a handheld measuring device according to one of Claims 1 to 9, **characterized in that** it comprises at least the following method steps:
a) collecting an interior sample,
b) accommodating the sample in a buffer,
c) immunological testing of the buffered sample
d) Evaluating the immunological test with electronic or optical sensors, and
e) displaying the test result from step d) in a control panel.

11. Method according to Claim 10, **characterized in that** collecting the sample is carried out in accordance with the impaction or filtration method.

12. Method according to Claim 10 or 11, **characterized in that** step a) and b), or step b) and c) are carried out simultaneously.

13. Method according to one of Claims 10 to 12, **characterized in that** the sample accommodated in a buffer in step b) is thoroughly mixed before the immunological testing in step c).

14. Method according to one of Claims 10 to 13, **characterized in that** the immunological testing in step c) tests for at least one component selected from the group of: ergosterol, beta-1,3-glucan, and the species-specific markers for mould fungi.

## Revendications

1. Appareil de mesure portable (1) permettant de détecter les dommages cachés dus aux moisissures, en particulier dans des locaux intérieurs,
**caractérisé en ce qu'**il présente au moins les composants suivants :
au moins une unité de collecte (2) qui reprend au moins un échantillon du local intérieur,
au moins une unité tampon (3),
au moins une unité (4) de test immunologique,
une unité d'évaluation (5) et
un panneau de commande (6).

2. Appareil de mesure portable (1) selon la revendication 1, **caractérisé en ce que** l'unité de collecte (2) présente une plaque de percussion et/ou un filtre.

3. Appareil de mesure portable (1) selon les revendications 1 ou 2, **caractérisé en ce que** l'unité de collecte (2) présente un module (8) d'aspiration d'air et/ou de poussière.

4. Appareil de mesure portable (1) selon l'une des revendications précédentes, **caractérisé en ce que** l'unité tampon (3) présente une cartouche tampon.

5. Appareil de mesure portable (1) selon l'une des revendications précédentes, **caractérisé en ce que** l'unité (4) de test immunologique présent des composants d'apport de réactif, de préférence une cartouche de réactif ou des composants de test à écoulement latéral.

6. Appareil de mesure portable (1) selon l'une des revendications précédentes, **caractérisé en ce que** l'unité d'évaluation (5) présente un module optique ou électronique de transduction.

7. Appareil de mesure portable (1) selon l'une des revendications précédentes, **caractérisé en ce que** la plaque de percussion et/ou le filtre, la cartouche tampon, la cartouche de réactif ou les composants de test à écoulement latéral sont réalisés en différentes combinaisons ou séparément comme composants à usage unique.

8. Appareil de mesure portable (1) selon l'une des revendications précédentes, **caractérisé en ce que** l'unité de collecte (2) et l'unité tampon (3) sont rassemblées en une entité.

9. Appareil de mesure portable (1) selon l'une des revendications précédentes, **caractérisé en ce qu'**une unité de mélange mécanique de l'échantillon est disposée entre l'unité tampon (3) et l'unité (4) de test immunologique.

10. Procédé de détection de dommages masqués dus aux moisissures, en particulier de dommages dus aux moisissures dans des locaux intérieurs, à l'aide d'un appareil de mesure portable selon l'une des revendications 1 à 9,
**caractérisé en ce qu'**il comporte au moins les étapes de procédé suivantes :
a) collecte d'un échantillon d'un local intérieur,
b) reprise de l'échantillon dans un tampon,
c) test immunologique de l'échantillon tamponné,
d) évaluation du test immunologique par des capteurs électroniques ou optiques et
e) affichage du résultat du test de l'étape d) sur un panneau de commande.

11. Procédé selon la revendication 10, **caractérisé en ce que** la collecte de l'échantillon est réalisée par un procédé d'impact ou de filtration.

12. Procédé selon les revendications 10 ou 11, **caractérisé en ce que** les étapes a) et b) ou les étapes b) et c) sont réalisées simultanément.

13. Procédé selon l'une des revendications 10 à 12, **caractérisé en ce que** l'échantillon repris dans un tampon à l'étape b) est mélangé avant le test immunologique de l'étape c).

14. Procédé selon l'une des revendications 10 à 13, **caractérisé en ce qu'**au moins un composant sélectionné dans l'ensemble constitué de l'ergostérol, du beta-1,3-glucane et de marqueurs spécifiques à des champignons et moisissures est testé à l'aide du test immunologique de l'étape c).
